# FASCICULE DE BREVET EUROPEEN

(11) **EP 1 223 177 B1**
(45) Date de publication et mention de la délivrance du brevet: **11.03.2009**
(21) Numéro de dépôt: 02290065.8
(22) Date de dépôt: 11.01.2002
(51) Int. Cl.: C07K 14/775, C12N 5/06, A01N 1/02

(54) **Procédé de production d'une fraction purifiée de lipoprotéines de faible densité de jaune d'oeuf et milieu de conservation incorporant de telles lipoprotéines**
Verfahren zur Herstellung einer gereinigten Fraktion an Low-Density-Lipoproteinen (LDL) aus Eigelb und Medium zur Konservierung, das diese Lipoproteine enthält
Process for the production of a purified fraction of low-density lipoproteins (LDL) from egg yolk and preservation medium containing such lipoproteins

(30) Priorité: 11.01.2001 FR 0100292
(43) Date de publication de la demande: 17.07.2002
(73) Titulaire: INSTITUT NATIONAL DE LA RECHERCHE AGRONOMIQUE (INRA), 75338 Paris Cédex 07 (FR); Ecole Nationale Vétérinaire de Nantes, 44307 Nantes Cedex 03 (FR)
(72) Inventeur: Anton, Marc, 44300 Nantes (FR); Martinet, Virginie, le Parc du Moulin des Roches, 44300 Nantes (FR); Tainturier, Daniel, 44470 Carquefou (FR); Moussa, Mohamed, 44307 Nantes Cédex 03 (FR)
(74) Mandataire: Laget, Jean-Loup

(56) Documents cités:
- DD-A- 273 164
- SU-A- 1 690 736
- US-A- 3 185 623
- US-A- 5 063 077
- OLLERO M ET AL.: "Loss of plasma membrane proteins of bull spermatozoa through the freezing-thawing process" THERIOGENOLOGY, vol. 49, no. 3, 1998, pages 547-555, XP001041502
- WEIDEL L ET AL.: "Cryosurvival of Human Spermatozoa Frozen in Eight Different Buffer Systems" JOURNAL OF ANDROLOGY, vol. 8, no. 1, 1 février 1987 (1987-02-01), pages 41-47, XP000576950
- DEMIANOWICZ W.; STRZEZEK J.: 'The effect of lipoprotein fraction from egg yolk on some of the biological properties of boar spermatozoa during storage of the semen in liquid state' REPRODUCTION ON DOMESTIC ANIMALS vol. 31, no. 1, 1996, pages 279 - 280
- BABIAK I. ET AL: 'The effect of egg yolk, low density lipoproteins, methylxanthines and fertilization diluent on cryopreservation efficiency of Northern pike spermatozoa' THERIOGENOLOGY vol. 52, 1999, pages 473 - 479

## Description

La présente invention concerne un procédé de production de fraction purifiée de lipoprotéines de faible densité (LDL - Low Density Lipoprotein) de jaune d'oeuf, un milieu de conservation de cellules vivantes contenant de telles lipoprotéines ainsi que l'utilisation de ces lipoprotéines de faible densité de jaune d'oeuf en tant qu'agent cryoprotecteur.

Le développement de technologies telles que l'insémination artificielle, la fécondation in vitro, la procréation assistée ont été facilitées par la possibilité de congeler des cellules vivantes, telles que des cellules reproductrices de type spermatozoïdes ou des cellules embryonnaires, dans des conditions permettant aux dites cellules de retrouver, après décongélation, leurs fonctionnalités.

Toutefois, pour améliorer les performances de ces méthodes, les techniques de congélation se sont elles-mêmes perfectionnées en vue notamment de réduire l'effet du choc thermique sur les cellules. Ainsi, tous les dilueurs de congélation de la semence incorporent aujourd'hui du jaune d'oeuf en tant qu'agent cryoprotecteur. Ce cryoprotecteur joue un rôle déterminant dans la protection des spermatozoïdes contre le choc thermique et il permet d'obtenir un plus grand nombre de spermatozoïdes vivants après décongélation et d'améliorer leur pouvoir fécondant. En revanche, le jaune d'oeuf est un bon milieu de culture pour tous les micro-organismes, ce qui augmente le risque de contamination bactérienne.

Des études ont donc été entamées ces dernières années en vue de remplacer le jaune d'oeuf contenu notamment dans les milieux de conservation de semence par un autre agent cyroprotecteur dont la nature est susceptible de limiter les contaminations microbiologiques. Toutefois, le mécanisme d'action du jaune d'oeuf, en tant qu'agent cryoprotecteur, n'étant à ce jour pas élucidé même si un certain nombre d'hypothèses ont été émises, ces études sont rendues difficiles.

Parallèlement, des essais ont été menés en vue d'isoler certains constituants du jaune d'oeuf, en particulier les lipoprotéines de faible densité du plasma de jaune d'oeuf dont elles forment le constituant principal. Toutefois, tous les procédés décrits à ce jour en la matière sont des procédés applicables uniquement en laboratoire et ne permettent pas une mise en oeuvre industrielle de tels procédés. Certaines méthodes ont en particulier été développées sur la base de techniques d'ultra-centrifugation qui nécessitent, pour l'isolement de lipoprotéines de faible densité de jaune d'oeuf, trente à quarante heures de centrifugation avec une ultra-centrifugeuse qui constitue un matériel cher non applicable à une mise en oeuvre industrielle.

Un tel exemple d'utilisation des techniques de centrifugation est décrit dans le brevet US-A-5.063.077. Le brevet DD273.164 décrit quant à lui l'utilisation de techniques d'ultrafiltration.

D'autres méthodes développées mettent en oeuvre des procédures chromatographiques à nouveau non applicables à l'échelle industrielle. Parallèlement, l'article d'OLLERO M et Al. : "Loss of plasma membrane proteins of bull spermatozoa through the freezing-thawing process" THERIOGENOLOGY, vol. 49, no. 3, 1998, pages 547-555 illustre une technique de séparation par électrophorèse des protéines incorporées à la membrane des spermatozoïdes à des fins d'analyse pour identifier le rôle des lipoprotéines en tant qu'agent cryoprotecteur. Toutefois, ce document ne décrit pas la préparation d'une telle fraction de lipoprotéines.

L'article de Demianowicz et Strzezek: "The effect of lipoprotein fraction from egg yolk on some of the biological properties of boar spermatozoa during storage of the semen in liquid state "Reproduction on domestic animals, vol, 31, no. 1 1996, pages 279-280 décrit l' utilisation de LDL de jaune d'oeuf en tant qu'un agent cryoprotecteur pour l'obtention d'un milieu de conservation des cellules vivantes.

Le but de la présente invention est donc de proposer un procédé d'obtention de fraction purifiée de lipoprotéines de faible densité de jaune d'oeuf à partir de plasma de jaune d'oeuf, ce procédé comportant des étapes susceptibles d'être mises en oeuvre de manière industrielle pour permettre une production en masse desdites lipoprotéines.

Un autre but de la présente invention est de proposer un milieu pour la conservation, à l'état réfrigéré ou congelé, de cellules vivantes, la composition de ce milieu permettant d'augmenter de manière significative le taux de survie et éventuellement la mobilité des cellules après décongélation.

A cet effet l'invention a pour objet un procédé de production de fraction purifiée de lipoprotéines de faible densité de jaune d'oeuf à partir de plasma de jaune d'oeuf, caractérisé en ce qu'il comprend au moins les étapes consistant :
- à additionner au moins un sel d'ammonium à du plasma de jaune d'oeuf en vue d'obtenir, après élimination du précipité formé, une fraction de plasma enrichie en lipoprotéines de faible densité de jaune d'oeuf et
- à dialyser la fraction de plasma enrichie en lipoprotéines de faible densité de jaune d'oeuf contre une solution aqueuse, de préférence saline, afin de provoquer, au cours de l'élimination des ions ammonium, une précipitation sélective des lipoprotéines de faible densité du jaune d'oeuf en vue de l'obtention d'une fraction purifiée de lipoprotéines de faible densité de jaune d'oeuf.

Le procédé décrit ci-dessus, qui repose sur une séparation des différentes parties du jaune d'oeuf par addition puis élimination d'au moins un sel suivie respectivement de centrifugations douces, est un procédé aisément extrapolable à l'échelle industrielle. L'étape clé de ce procédé réside dans l'étape de dialyse au cours de laquelle les inventeurs ont constaté que, de manière surprenante, l'utilisation d'un milieu de dialyse constitué essentiellement d'eau permettait d'obtenir une précipitation sélective des lipoprotéines de faible densité du plasma enrichi en lipoprotéines de faible densité de jaune d'oeuf. Cette précipitation sélective des lipoprotéines de faible densité au cours de l'élimination des ions ammonium n'avait jamais été constatée à ce jour.

L'invention a encore pour objet un milieu pour la conservation, à l'état réfrigéré ou congelé, de cellules vivantes, telles que de la semence ou un embryon, le milieu étant constitué d'un milieu de base formé de constituants, tels qu'un agent de nutrition et un antibiotique, convenant pour la conservation desdites cellules, caractérisé en ce qu'il comprend en outre, comme agent cryoprotecteur des cellules, une fraction purifiée de lipoprotéines de faible densité (LDL) de jaune d'oeuf pour limiter, de manière significative, l'effet du choc thermique sur les caractéristiques fonctionnelles desdites cellules, cette fraction purifiée de lipoprotéines de faible densité (LDL) de jaune d'oeuf pour limiter, de manière significative, l'effet du choc thermique sur les caractéristiques fonctionnelles desdites cellules, cette fraction purifiée de lipoprotéines de faible densité de jaune d'oeuf, obtenue selon le procédé du type décrit ci-dessus présentant un taux de pureté au moins égal à 97 %.

Des études effectuées à partir de ce milieu ont montré que les lipoprotéines de faible densité possèdent un effet cryoprotecteur supérieur aux lipoprotéines incluses dans du jaune d'oeuf complet, notamment sur de la semence de taureau réfrigérée ou congelée et ce de manière inattendue. Cet effet supérieur s'apprécie par la mesure du taux de survie et la mobilité des spermatozoïdes après mise en oeuvre du choc thermique.

L'invention a encore pour objet l'utilisation d'une fraction purifiée de lipoprotéines de faible densité de jaune d'oeuf présentant un taux de pureté au moins égal à 97 % en tant qu'agent cryoprotecteur pour l'obtention d'un milieu de conservation de cellules vivantes à l'état réfrigéré ou congelé.

L'invention a encore pour objet l'utilisation d'un milieu de conservation du type précité pour la conservation à l'état réfrigéré ou congelé de sperme de vertébrés en vue de l'insémination artificielle.

L'invention sera bien comprise à la lecture de la description suivante d'exemples de réalisation, en référence aux dessins annexés dans lesquels :
la figure 1 représente de manière schématique les différentes étapes du procédé d'obtention de fraction de lipoprotéines de faible densité de jaune d'oeuf ;
la figure 2 représente un histogramme montrant l'effet des différentes concentrations de LDL du jaune d'oeuf de poule sur la mobilité des spermatozoïdes de taureau après conservation à +4°c pendant 24 heures et
la figure 3 représente l'effet de différentes concentrations de LDL du jaune d'oeuf de poule sur la mobilité des spermatozoïdes de taureau après congélation et décongélation.

Comme mentionné ci-dessus, les seuls méthodes existant à ce jour pour purifier les lipoprotéines de faible densité (LDL) de jaune d'oeuf sont des méthodes de laboratoire qui présentent un faible rendement et mettent en jeu des procédés non-extrapolables à l'échelle industrielle. Ces méthodes reposent notamment sur des étapes d'ultra-centrifugation et de chromatographie d'exclusion stérique.

Les lipoprotéines faible densité (LDL) forment le constituant principal du plasma de jaune d'oeuf. Ces lipoprotéines sont organisées en sphères de 17 nm à 60 nm de diamètre, ces sphères étant elles-mêmes constituées d'un noyau de lipides neutres (triglycérides et ester de cholestérol) entouré par un film de phospholipides et de protéines en contact avec la phase aqueuse. Ces LDL, qui sont les constituants majeurs du jaune d'oeuf, contiennent 83 - 89 % de lipides et 11 - 17 % de protéines. Elles ont un poids moléculaire compris entre 1.10⁶ Da et 3.10⁶ Da. Les lipides se répartissent quant à eux en 74 % de lipides neutres (triglycérides et cholestérol) et en 26 % de phospholipides. Il est connu depuis longtemps que les lipoprotéines de faible densité sont contenues dans le plasma de jaune d'oeuf.

La première étape de tout procédé de production d'une fraction purifiée de lipoprotéines de faible densité de jaune d'oeuf consiste donc à séparer, dans le jaune d'oeuf, le plasma des granules.

Bien évidemment, préalablement à la séparation du plasma des granules, il convient tout d'abord d'extraire le jaune d'oeuf de l'oeuf. Pour ce faire, il est procédé à un cassage d'oeufs, ces oeufs étant généralement issus de poules élevées dans un élevage fermier. Ces oeufs doivent être cassés au maximum 24 heures après la ponte. Le jaune est séparé du blanc. Chaque jaune est roulé délicatement sur du papier filtre pour éliminer l'albumen et les chalazes qui le relient à la membrane vitelline. La membrane est ensuite incisée par une lame de scalpel et le jaune est déversé dans une éprouvette maintenue dans de la glace pilée pour éviter la dénaturation des protéines. Le jaune présente une teneur en matières sèches d'environ 52,5 %. Bien évidemment, cette étape de cassage d'oeufs et de séparation du jaune du blanc d'oeuf pourra être modifiée pour sa mise en oeuvre à l'échelle industrielle.

Pour permettre ensuite la séparation du plasma et des granules, le jaune pur est dilué dans une solution de chlorure de sodium (NaCl 0,17 M dans un rapport de 1:1 poids : poids) puis est soumis à une agitation modérée à 4°C durant 1 heure sur un agitateur magnétique permettant d'équilibrer la solution. Cette dilution est ensuite centrifugée à 10000g pendant 45 minutes à une température de 4°C. Le surnageant liquide constituant le plasma est prélevé et recentrifugé dans les mêmes conditions. Le plasma purifié est récupéré et conservé à 4°C. Le culot de centrifugation constitué des granules est quant à lui éliminé. Cette étape de séparation du plasma des granules est une étape classique bien connue à ceux versés dans cet art.

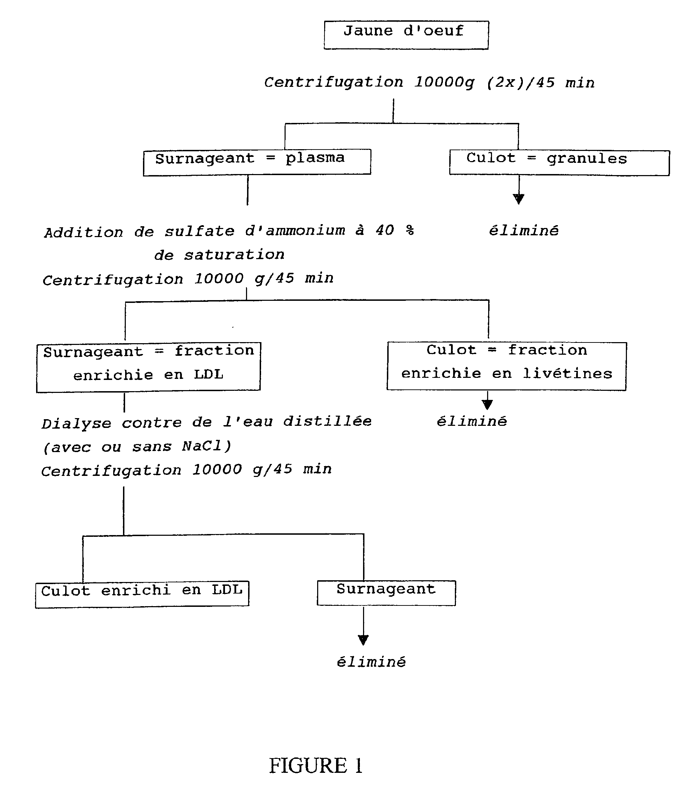

La seconde étape consiste à obtenir une fraction de plasma enrichie en lipoprotéines de faible densité, notamment en éliminant du plasma les livétines contenues dans ce plasma. Pour ce faire, on additionne au moins un sel d'ammonium au plasma de jaune d'oeuf en vue d'obtenir, après élimination du précipité formé, une fraction enrichie en lipoprotéines de faible densité de jaune d'oeuf. L'addition de sel d'ammonium s'effectue à froid en maintenant le pH constant proche de 8,7. Le sel d'ammonium utilisé est de préférence du sulfate d'ammonium à au moins 30 % de saturation. Dans le procédé illustré à la figure 1, le sulfate d'ammonium utilisé est un sulfate d'ammonium à 40 % de saturation, soit 20,5 g de sulfate d'ammonium pour 100 ml de plasma. Le sulfate d'ammonium doit être ajouté très lentement afin d'éviter la dénaturation des protéines. Une agitation d'une heure est ensuite nécessaire pour équilibrer la solution. Cette agitation s'effectue à 4°C. Le mélange est ensuite centrifugé à 10000g pendant 45 minutes, ce qui permet d'obtenir un culot et un surnageant. Le surnageant correspond à la fraction de plasma enrichie en lipoprotéines de faible densité de jaune d'oeuf tandis que le culot correspond à la fraction de plasma enrichie en livétines. Ce culot de centrifugation sera éliminé, tandis que le surnageant va ensuite être soumis à une étape de dialyse.

La dialyse est effectuée sur une durée de dix heures environ. Le bain de dialyse est constitué d'eau distillée contenant de 0 à 50 mM de NaCl. Ce bain de dialyse est changé toutes les deux heures et une agitation constante est assurée. Ainsi, dans l'exemple représenté à la figure 1, 200 ml de surnageant sont dialysés contre 7,51 d'eau salée pendant 10 heures avec cinq changements de bain de dialyse. La concentration de NaCl est augmentée à chaque changement de bain de dialyse pour éviter une destruction des micelles des lipoprotéines. Le membrane utilisée est une membrane MCL 8x100 CLR. La membrane est mise à bouillir dans un bain-marie pendant 15 minutes préalablement à la dialyse. Au cours de la dialyse, l'élimination des ions ammonium provoque une précipitation sélective d'un culot riche en lipoprotéines de faible densité. Après dialyse, le mélange est centrifugé à 10000g pendant 45 minutes et un culot de lipoprotéines de faible densité pur à 97 % est obtenu et conservé à 4°C.

Le procédé décrit ci-dessus permet ainsi d'obtenir en 24 heures environ 9 g de culot à partir de 16ml de jaune d'oeuf. Comme mentionné ci-dessus, ce culot présente des caractéristiques très proches de celles des lipoprotéines de faible densité pures avec un degré de pureté au moins égal à 97 % et proche en réalité de 97,3 % permettant d'envisager une production industrielle. Par ailleurs, le rendement d'un tel procédé est particulièrement bon, au moins égal à 60 %.

A partir de ces fractions purifiées de lipoprotéines de faible densité de jaune d'oeuf obtenues, des essais ont été réalisés en vue de démontrer que l'utilisation de ces fractions dans des milieux utiles pour la conservation, à l'état réfrigéré ou congelé, de cellules vivantes permettait d'obtenir des résultats inattendus.

Ainsi, les essais ont montré qu'un milieu comprenant en sus d'un milieu de base classique formé de constituants, tels qu'un agent de nutrition et un antibiotique, convenant pour la conservation des cellules vivantes, une fraction de lipoprotéines de faible densité de jaune d'oeuf comme agent cryoprotecteur des cellules en remplacement du jaune d'oeuf, permettait d'obtenir une augmentation significative de la durée de vie et de la mobilité des cellules en particulier lorsque ces cellules sont constituées par des spermatozoïdes.

Il a été montré que cette activité du milieu de conservation était plus particulièrement obtenue lorsque la fraction de lipoprotéines de faible densité de jaune d'oeuf est présente dans le milieu de conservation à une concentration P/V (poids/volume) comprise dans la plage [2,5 % - 15 %] et de préférence dans la plage [5 % - 10 %].

L'exemple décrit ci-dessous s'applique à un milieu de conservation dans lequel le milieu de base est un dilueur de congélation constitué notamment d'au moins un antibiotique, d'un agent de nutrition et d'un tampon, ce milieu de base convenant pour la dilution de sperme d'une espèce animale déterminée.

Le milieu de base retenu est constitué par le milieu Triladyl classiquement utilisé comme dilueur de congélation. La composition de ce milieu de base est telle que suit : 100ml de ce milieu Triladyl contient 2,42 g de Tris, 1,48g d'acide citrique, 1,00g de fructose, 6,4 ml de glycérol. La concentration par ml d'antibiotique est de 250µg de gentamicine et de 150µg de penicilline.

A partir de ce milieu de base, sept milieux de conservation ont été préparés comme l'illustre le tableau qui suit.

| | | | | | | |
|---|---|---|---|---|---|---|
| Milieu Témoin | LDL1 2,5 % | LDL2 5 % | LDL3 10 % | LDL4 15 % | LDL5 20 % | Milieu Biociphos |
| Milieu de base | Milieu de base | Milieu de base | Milieu de base | Milieu de base | Milieu de base | composition inconnue |
| Jaune d'oeuf 20 ml | LDL 2,5% | LDL 5 % | LDL 10 % | LDL 15 % | LDL 20 % | |

Dans ce tableau, le milieu Triladyl contenant 20 ml de jaune d'oeuf et le milieu Biociphos commercialisé par la société Instruments de Médecine Vétérinaire 61302, L'Aigle, France sont utilisés comme milieux témoins. Il est à noter que la composition du milieu Biociphos est inconnue. La seule donnée relative à ce milieu concerne l'utilisation d'agent cryoprotecteur à base de lécithine de soja. Parallèlement à ces deux milieux témoins, cinq milieux LDL1 à LDL5 sont préparés. Chaque milieu est constitué du milieu de base (Triladyl) et d'une fraction de lipoprotéines de faible densité de jaune d'oeuf à des concentrations (P/V) comprises entre 2,5 et 20 %. Bien évidemment, cette fraction de lipoprotéines de faible densité est de préférence obtenue conformément au procédé précité.

A partir de ces milieux, deux expériences ont été menées de manière à démontrer l'influence de la fraction LDL du jaune d'oeuf à différentes concentrations au cours de la conservation de spermatozoïdes de taureau à l'état réfrigéré et à l'état congelé.

Expérience n° 1 : Des éjaculats récoltés sont dilués dans les milieux de conservation préparés ci-dessus. Il est procédé de la manière suivante. Sept tubes à essai sont placés au bain marie à 34°C, chaque tube reçoit 0,5 ml de l'un des milieux précités et 0,5 ml de sperme pour obtenir un volume final de 1 ml. Les tubes sont laissés 10 minutes à 34°C. Pendant ce temps la concentration en spermatozoïdes est calculée de façon à rediluer le mélange pour obtenir une concentration finale de 120.10⁶ spermatozoïdes par ml.

Un ml de chaque tube renfermant 120.10⁶ spermatozoïdes par ml est alors placé dans un cryotube et conservé pendant 24 heures à +4°C avant d'être analysé. Le système d'analyse retenu est le système ATS 20 (J.C.

Diffusion Internationale, La Ferté Fresnet, France).

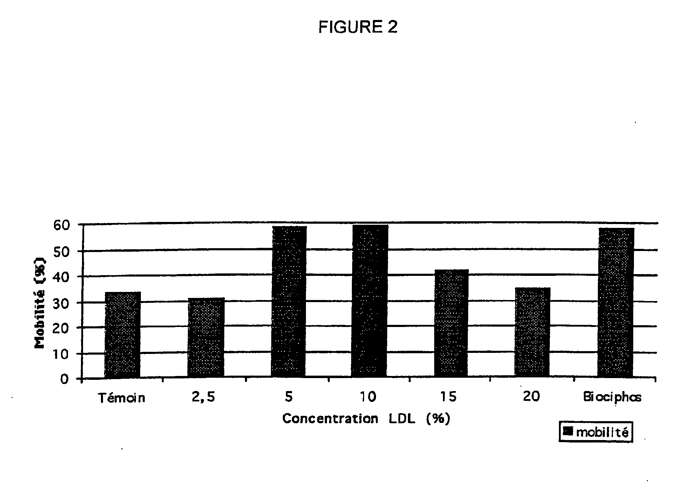

Les résultats sont fournis à la figure 2 dans laquelle il est représenté en abscisses chaque milieu de conservation précité et en ordonnées la mobilité des spermatozoïdes dans ledit milieu. Ces résultats montrent clairement l'effet bénéfique de la fraction LDL de jaune d'oeuf sur la mobilité des cellules à une concentration P/V comprise dans la plage (5% - 10%).

Expérience N° 2 : Après cette descente de température en une heure trente à +4°C, cinq paillettes de 0,5 ml sont préparées à partir de chacun des sept tubes. Les sept lots de cinq paillettes sont maintenus à +4°C pour l'équilibration pendant deux heures puis ils sont congelés. La congélation est réalisée dans les vapeurs d'azote liquide en descendant progressivement la rampe supportant les paillettes vers l'azote liquide. La sonde d'un thermocouple placée sur la rampe permet de contrôler la courbe de refroidissement. Lorsque la température atteint -150°C, les paillettes sont plongées dans l'azote liquide à -196°C. Le stockage s'effectue en cuve, dans l'azote liquide, à -196°C.

Pour permettre la décongélation des paillettes ainsi congelées, trois paillettes de chaque concentration de la fraction LDL du jaune d'oeuf sont immergées pendant 45 secondes dans un bain-marie à 37°C. A l'issue de cette décongélation, chaque paillette est essuyée, ses extrémités sont sectionnées avec une paire de ciseaux puis vidées dans un cryotube placé au bain thermostaté à 37°C. Après être restée 10 minutes à 37°C, la semence est analysée à l'aide du système ATS 20.

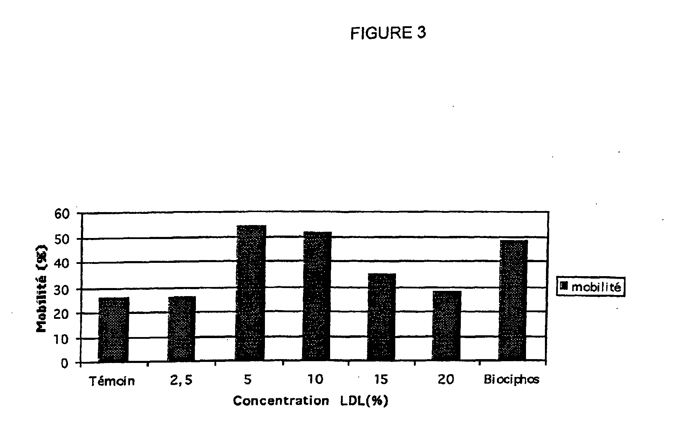

Les résultats sur la mobilité des spermatozoïdes de taureau après congélation et décongélation sont illustrés dans la figure 3 dans laquelle il est représenté en abscisse chaque milieu de conservation précité et en ordonnées la mobilité des spermatozoïdes dans ledit milieu.

On observe ainsi que la mobilité des spermatozoïdes augmente à partir d'une concentration de 2,5 % de lipoprotéines de faible densité de jaune d'oeuf dans le milieu de base et atteint un maximum entre 5 et 10 % de lipoprotéines de faible densité, puis commence à diminuer à partir de 15 % de lipoprotéines de faible densité par rapport au témoin quelles que soient les conditions de refroidissement.

Ces résultats montrent clairement que la fraction lipoprotéines de faible densité possède un effet bénéfique sur la mobilité et les caractéristiques des mouvements des spermatozoïdes réfrigérés à +4°C ou congelés par rapport au jaune d'oeuf non traité contenu dans le témoin. Il est permis de s'étonner de ces résultats sachant que le jaune d'oeuf renferme l'équivalent de 6,6 % de lipoprotéines de faible densité.

## Revendications

1. Procédé de production de fraction purifiée de lipoprotéines de faible densité de jaune d'oeuf à partir de plasma de jaune d'oeuf,
**caractérisé en ce qu'**il comprend au moins les étapes consistant :
- à additionner au moins un sel d'ammonium à du plasma de jaune d'oeuf en vue d'obtenir, après élimination du précipité formé, une fraction de plasma enrichie en lipoprotéines de faible densité de jaune d'oeuf et
- à dialyser la fraction de plasma enrichie en lipoprotéines de faible densité de jaune d'oeuf contre une solution aqueuse, de préférence saline, afin de provoquer, au cours de l'élimination des ions ammonium, une précipitation sélective des lipoprotéines de faible densité de jaune d'oeuf en vue de l'obtention d'une fraction purifiée de lipoprotéines de faible densité de jaune d'oeuf.

2. Procédé selon la revendication 1,
**caractérisé en ce qu'**on effectue l'addition du sel d'ammonium, constitué d'un sulfate d'ammonium à au moins 30 % de saturation, à froid en maintenant le pH constant proche de 8,7, **en ce qu'**on équilibre sous agitation la solution formée et **en ce qu'**on centrifuge ladite solution pour permettre de fractionner le plasma de jaune d'oeuf en un culot et en un surnageant, le surnageant, correspondant à la fraction de plasma enrichi en lipoprotéines de faible densité de jaune d'oeuf, étant soumis ultérieurement à une étape de dialyse.

3. Procédé selon l'une des revendications 1 et 2,
**caractérisé en ce qu'**on effectue la dialyse sur une durée de dix heures environ, le bain de dialyse constitué d'eau contenant de 0 à 50 mM de NaCl étant changé environ toutes les deux heures et **en ce qu'**on soumet, après dialyse, le mélange à une centrifugation douce pour obtenir un culot de centrifugation constitué de lipoprotéines de faible densité pur à au moins 97 %.

4. Milieu pour la conservation à l'état réfrigéré ou congelé de cellules vivantes telles que de la semence ou un embryon, le milieu étant constitué d'un milieu de base formé de constituants, tels qu'un agent de nutrition et un antibiotique, convenant pour la conservation desdites cellules,
**caractérisé en ce qu'**il comprend en outre, comme agent cryoprotecteur des cellules, une fraction purifiée de lipoprotéines de faible densité (LDL) de jaune d'oeuf pour limiter, de manière significative, l'effet du choc thermique sur les caractéristiques fonctionnelles desdites cellules, cette fraction purifiée de lipoprotéines de faible densité de jaune d'oeuf, obtenue selon le procédé conforme à l'une des revendications 1 à 3, présentant un taux de pureté au moins égal à 97 %.

5. Milieu de conservation selon la revendication 4,
**caractérisé en ce que** la fraction de lipoprotéines de faible densité de jaune d'oeuf est présente dans le milieu à une concentration P/V comprise dans la plage [2,5 % - 15 %] et de préférence dans la plage [5 % - 10 %].

6. Milieu de conservation selon l'une des revendications 4 et 5,
**caractérisé en ce que** le milieu de base est un dilueur de congélation convenant pour la dilution de sperme d'une espèce animale déterminée, ce dilueur constitué notamment d'au moins un antibiotique, un agent de nutrition et un tampon, renfermant en outre en tant qu'agent cryoprotecteur une fraction de lipoprotéines de faible densité de jaune d'oeuf.

7. Milieu de conservation selon l'une des revendications 4 à 6,
**caractérisé en ce que** le jaune d'oeuf est un jaune d'oeuf de poule.

8. Utilisation d'une fraction purifiée de lipoprotéines de faible densité de jaune d'oeuf obtenue selon le procédé conforme à l'une des revendications 1 à 3, présentant un taux de pureté au moins égal à 97 % en tant qu'agent cryoprotecteur pour l'obtention d'un milieu de conservation de cellules vivantes à l'état réfrigéré ou congelé.

9. Utilisation d'un milieu de conservation selon l'une des revendications 4 à 7, pour la conservation à l'état réfrigéré ou congelé de sperme de vertébrés en vue de l'insémination artificielle.

## Claims

1. A process for the production of a purified fraction of egg yolk low-density lipoproteins from egg yolk plasma,
**characterised in that** it comprises at least the steps consisting of:
- adding at least one ammonium salt to egg yolk plasma so as to obtain, after eliminating the precipitate formed, a plasma fraction enriched in egg yolk low-density lipoproteins and
- dialysing the plasma fraction enriched in egg yolk low-density lipoproteins against an aqueous, preferably saline, solution in order to bring about, during the elimination of the ammonium ions, a selective precipitation of the egg yolk low-density lipoproteins so as to obtain a purified fraction of egg yolk low-density lipoproteins.

2. The process according to claim 1,
**characterised in that** the addition of the ammonium salt, made up of an at least 30% saturated ammonium sulfate, is carried out cold, keeping the pH constant at about 8.7, **in that** the solution formed is equilibrated while stirring and **in that** said solution is centrifuged to enable the egg yolk plasma to be fractionated into a pellet and a supernatant, the supernatant, corresponding to the plasma fraction enriched in egg yolk low-density lipoproteins, being subsequently subjected to a dialysis step.

3. The process according to one of claims 1 and 2,
**characterised in that** the dialysis is carried out over a period of approximately ten hours, the dialysis bath made up of water containing 0 to 50 mM of NaCl being changed approximately every two hours, and **in that**, after dialysis, the mixture is subjected to gentle centrifugation to obtain a centrifugation pellet made up of low-density lipoproteins with a purity of at least 97%.

4. A medium for the preservation in the refrigerated or frozen state of living cells, such as semen or an embryo, the medium being made up of a basic medium formed by constituents such as a nutrition agent and an antibiotic, suitable for the preservation of said cells,
**characterised in that** it also comprises, as a cell cryoprotectant, a purified fraction of egg yolk low-density lipoproteins (LDL) in order to limit significantly the effect of thermal shock on the functional characteristics of said cells, this purified fraction of egg yolk low-density lipoproteins, obtained by the process according to one of claims 1 to 3, having a level of purity at least equal to 97%.

5. The preservation medium according to claim 4,
**characterised in that** the egg yolk low-density lipoprotein fraction is present in the medium in a w/v concentration in the range of [2.5% - 15%] and preferably in the range of [5% - 10%].

6. The preservation medium according to one of claims 4 and 5,
**characterised in that** the basic medium is a freezing diluter suitable for diluting the sperm of a specific animal species, this diluter being made up in particular of at least one antibiotic, a nutrition agent and a buffer, additionally including as cryoprotectant an egg yolk low-density lipoprotein fraction.

7. The preservation medium according to one of claims 4 to 6,
**characterised in that** the egg yolk is a hen's egg yolk.

8. The use of a purified fraction of egg yolk low-density lipoproteins obtained by the process according to one of claims 1 to 3, having a level of purity at least equal to 97%, as a cryoprotectant in order to obtain a medium for the preservation of living cells in the refrigerated or frozen state.

9. The use of a preservation medium according to one of claims 4 to 7 for the preservation in the refrigerated or frozen state of vertebrate sperm for the purpose of artificial insemination.

## Patentansprüche

1. Verfahren zur Herstellung einer gereinigten Fraktion von Lipoproteinen niedriger Dichte aus Eigelb ausgehend von Eigelbplasma, **dadurch gekennzeichnet, dass** es mindestens die Schritte umfasst, die darin bestehen,
- dem Eigelbplasma mindestens ein Ammoniumsalz hinzuzufügen, um nach der Entfernung des gebildeten Niederschlags eine Fraktion von mit Lipoproteinen niedriger Dichte aus Eigelb angereichertem Plasma zu erhalten, und
- die Fraktion von mit Lipoproteinen niedriger Dichte aus Eigelb angereichertem Plasma gegen eine wässrige Lösung, vorzugsweise eine Salzlösung, zu dialysieren, um während der Entfernung der Ammoniumionen eine selektive Fällung der Lipoproteine niedriger Dichte aus Eigelb herbeizuführen und somit eine gereinigte Fraktion von Lipoproteinen niedriger Dichte aus Eigelb zu erhalten.

2. Verfahren nach Anspruch 1, **dadurch gekennzeichnet, dass** die Hinzufügung des Ammoniumsalzes, das aus einem Ammoniumsulfat mit mindestens 30 % Sättigung besteht, im kalten Zustand unter Beibehaltung eines konstanten pH-Wertes um 8,7 erfolgt, dass die gebildete Lösung unter Bewegung gemischt wird und dass die Lösung zentrifugiert wird, um das Eigelbplasma in einen Rückstand und in einen Überstand zu fraktionieren, wobei der Überstand, der der Fraktion des mit Lipoproteinen niedriger Dichte aus Eigelb angereicherten Plasmas entspricht, später einer Dialyse unterzogen wird.

3. Verfahren nach einem der Ansprüche 1 und 2, **dadurch gekennzeichnet, dass** die Dialyse über eine Dauer von etwa zehn Stunden erfolgt, wobei die Dialyseflüssigkeit, die aus Wasser mit einem NaCl-Gehalt von 0 bis 50 mM besteht, etwa alle zwei Stunden ausgetauscht wird, und dass das Gemisch nach der Dialyse leicht zentrifugiert wird, um einen Zentrifugenrückstand zu erhalten, der aus zu mindestens 97 % reinen Lipoproteinen niedriger Dichte besteht.

4. Medium für die Konservierung lebender Zellen im gekühlten oder gefrorenen Zustand, wie Samen oder Embryonen, das aus einem Grundmedium besteht, das aus Bestandteilen wie einem Nährmittel und einem Antibiotikum besteht, die für die Konservierung der Zellen geeignet sind, **dadurch gekennzeichnet, dass** es zudem, als Kälteschutzmittel für die Zellen, eine gereinigte Fraktion von Lipoproteinen niedriger Dichte (LDL) aus Eigelb umfasst, um die Wirkung des thermischen Schocks auf die funktionalen Merkmale der Zelle erheblich zu begrenzen, wobei die gereinigte Fraktion von Lipoproteinen niedriger Dichte aus Eigelb, die mit dem Verfahren nach einem der Ansprüche 1 bis 3 erhalten wurde, einen Reinheitsgrad von mindestens 97 % aufweist.

5. Konservierungsmedium nach Anspruch 4, **dadurch gekennzeichnet, dass** die Fraktion von Lipoproteinen niedriger Dichte aus Eigelb in dem Medium in einer Konzentration (Gewicht/Volumen) im Bereich von [2,5 bis 15 %] und vorzugsweise im Bereich von [5 % bis 10 %] vorhanden ist.

6. Konservierungsmedium nach einem der Ansprüche 4 und 5, **dadurch gekennzeichnet, dass** das Grundmedium ein Gefrierverdünner ist, der für die Verdünnung des Samens einer bestimmten Tierart geeignet ist, wobei der Verdünner, der insbesondere mindestens aus einem Antibiotikum, einem Nährmittel und einem Puffer besteht, als Kälteschutzmittel zudem eine Fraktion von Lipoproteinen niedriger Dichte aus Eigelb einschließt.

7. Konservierungsmedium nach einem der Ansprüche 4 bis 6, **dadurch gekennzeichnet, dass** das Eigelb ein Hühnereigelb ist.

8. Verwendung einer gereinigten Fraktion von Lipoproteinen niedriger Dichte aus Eigelb, die mit dem Verfahren nach einem der Ansprüche 1 bis 3 erhalten wurde und einen Reinheitsgrad von mindestens 97 % aufweist, als Kälteschutzmittel für den Erhalt eines Mediums für die Konservierung lebender Zellen im gekühlten oder gefrorenen Zustand.

9. Verwendung eines Konservierungsmediums nach einem der Ansprüche 4 bis 7 für die Konservierung im gekühlten oder gefrorenen Zustand von Wirbeltiersamen für die künstliche Besamung.
